# EUROPEAN PATENT APPLICATION

(11) **EP 4 748 354 A1**
(43) Date of publication of application: **27.05.2026**
(21) Application number: 24315528.0
(22) Date of filing: 22.11.2024
(51) Int. Cl.: A61F 5/00, A61F 2/04, A61F 2/95, A61F 2/966, A61B 1/00, A61B 17/221

(54) **MEDICAL DEVICE, IN PARTICULAR AN ENDOSCOPE AND/OR A DELIVERY DEVICE, AND ASSOCIATED METHODS**

(71) Applicant: BariaTek Medical, 75013 Paris (FR)
(72) Inventor: GRAY, Yonatan, 75011 Paris (FR); BAERD, Leonard Raphaël, 75018 Paris (FR); NAZ, Christophe, 77300 Fontainebleau (FR); BIADILLAH, Youssef, 78600 Maisons-Laffitte (FR)
(74) Representative: Hepp Wenger Ryffel AG

(57) **Abstract**

The invention is directed to a medical device (10) comprising an elongated main shaft (40) and an attachment element for attaching to a line arranged on an outside of the elongated main shaft. The attachment element being positioned at or in vicinity of a distal portion of the elongated main shaft.

## Description

The invention relates to a medical device, e.g. for introduction and/or for the delivery of an implant within the gastrointestinal tract of a patient. The invention is further directed to a method of implanting and/or delivering an implant within the gastrointestinal tract of a patient.

Delivery systems for delivery of implants in a patient's gastro-intestinal tract are known in the prior art. However technical difficulties remain in the delivering of implants within a gastrointestinal tract. Such systems have drawbacks related to their ease of use, and the difficulties relating to the accessibility of the intestine for the delivery of bypass sleeves. The distance that can be navigated by a delivery system is often limited by practical considerations such as size, friction of the device with intestinal tissue, ease of navigation with minimised risk of damage to surrounding tissue. For example, high pushing forces used to navigate the delivery system may risk damaging intestinal tissue, and can lead to perforation of a gastrointestinal wall.

EP 1 768 618 discloses a delivery system for placing a gastrointestinal implant in a gastrointestinal tract. The system comprises a container assembly and a gastrointestinal implant device. The implant device has a proximal end and a distal end, and the proximal end includes an anchor. The distal end includes a sleeve. The proximal end and the distal end are stored within the container assembly of the delivery system.

EP 3 407 804 discloses a delivery system for delivering a gastrointestinal implant in a gastrointestinal tract. The delivery system includes a delivery catheter including a lumen; a container assembly at the distal portion of the delivery catheter, the container assembly including a proximal capsule and a distal cap removably attachable thereto; and a gastrointestinal implant.

US 2006/155312 discloses a delivery system comprising an outer sheath, or container, for storing a proximal portion of the implant device, and an inner sheath within the outer sheath, and an atraumatic feature such as a ball coupled to the distal end of the inner sheath to guide the delivery system through the alimentary canal.

The devices according to the prior art all have certain disadvantages. For instance, some may require the insertion of both the delivery system and an endoscope in parallel to one another, this leads to a bulky ensemble which may lead to injury of the patient. Others rely on a sequential insertion of the endoscope and the delivery system and follow a process called "guidewire exchange" wherein an endoscope is first inserted into the patients' gastrointestinal tract. A guidewire is then fed along the endoscope into the gastrointestinal tract. The endoscope is then removed and the delivery system is inserted over the guidewire into the gastrointestinal tract. This process leads to prolonged intervention times, which may increase the risk to patient safety. Also, the sequential introduction of the endoscope and delivery system can lead to reduced visualisation of the process of delivering the implant during the procedure. Low visibility may in turn result in incorrect positioning of an implant, or insufficient anchoring of the implant. The incorrect delivery of the implant will only be noticed after reinsertion of the endoscope after removal of the delivery system and can lead to the operator having to essentially restart the process.

It is an objective of the present invention to overcome the drawbacks of the prior art, in particular to provide medical devices and in particular gastrointestinal delivery systems that are easier and less time consuming in their use as well as improving the implant delivery process.

A further objective of the invention is to provide a delivery system which provides for a more streamlined procedure and easier deployment and reliable anchoring of the implant within the gastrointestinal tract of the patient.

These and other objectives are achieved with systems, assembly and methods according to the independent claims. Further embodiments result from the dependant claims.

The invention is directed to a medical device. The medical device may in particular be a delivery system for introducing and/or delivering an implant within the gastro-intestinal tract, and/or the medical device may be an endoscope. The medical device comprises an elongated main shaft. Furthermore, an attachment element, e.g. a snare, is provided for attaching to a line arranged on an outside of the elongated main shaft. The attachment element is positioned at or in the vicinity of a distal portion of the elongated main shaft.

The attachment element may directly or indirectly attach to the medical device. For example, the attachment element may indirectly attach to the medical device by attaching to a component attached to the medical device (e.g. an implant) or section of the medical device such as the elongate main shaft.

Thus, the main shaft may be oriented by pulling with the line. This avoids the need for separate devices for positioning and orienting, such as guidewires. As a result, the device may be more compact, thus providing more space and allowing the use of other instruments where necessary.

The attachment element may be configured to at least partly attached to any one or more of: the elongated shaft, preferably the distal end of the elongated main shaft; an implant holder; an implant; an implant sheath for positioning of an implant at a target site. Additionally or alternatively, the attachment element can be configured to at least partly encircle any one or more of: the elongated shaft, preferably the distal end of the elongated main shaft; an implant holder; an implant; an implant sheath for positioning of an implant at a target site.

The attachment element in some embodiments directly or indirectly connects a distal end of a flexible line to an implant. The flexible line may be provided as any one or more of: a cable, a wire, a tube, or a filament. The attachment element may include any one or more of: a loop; a band; a ring; a mesh; a wire; a filament a cable or a tube.

Additional or alternatively, the attachment element may include any one or more of a gripper; an adhesive; a clamp; a clip. For instance, the attachment element may be a gripper or snare configured to grip or hold an extraction mechanism or a connection element of the implant sheath.

In a preferred embodiment, the attachment element is a snare. The snare may be formed by a tubular flexible line and a looped attachment element. The flexible line may be integrally formed with the attachment element. The snare may include a flexible plastic tube housing a wire.

The attachment element, in particular the snare, may be configured to at least partly cause the elongated main shaft to bend from a first position to a second position for positioning the implant at a target site. The bending of the elongated main shaft may be achieved by a translational movement of the attachment element, such as a pulling motion applied to the flexible line having the attachment element at its distal end. It is conceivable that the attachment element may bend the elongated shaft by a pushing motion. The attachment element may provide for a more precise positioning of the implant at the target site.

To achieve this, the flexible line and/or an attachment element, is at least partly bendable. The translation of the attachment element, preferably of the flexible line, by pushing or pulling induces a movement of the implant or elongated main shaft attached to the flexible line via the attachment element.

As a first example, the attachment element can be provided as a lasso which encircles a portion of an implant sheath, thus grasping the sheath. The implant sheath securely retains at least a portion of the implant until such time as an extraction mechanism of the sheath is operated. As the implant is coupled to the delivery system via the implant holder, pulling the attachment element will thus induce tugging of the extraction mechanism of the sheath which in turn causes the elongated shaft to bend and moves the implant. The attachment element of this example may also serve to actuate the extraction mechanism of the sheath. This may be possible for instance, by applying a greater pulling force on the attachment element as compared to the force applied when repositioning the implant.

The same principle applies when the attachment element is attached to and/or encircles any one of: the distal end of the shaft, the implant holder or the implant.

In particular, the attachment element and the flexible line reduces the amount of instruments needed as for recovery of the sheath as well as for the positioning of the implant and therefore allow for a more compact easy to use device. Furthermore, they may help to reduce the duration of the intervention as it may require less manipulation time by the operator as compared to conventional methods.

Advantageously, the flexible line and the attachment element may help reposition the elongated shaft within the gastrointestinal tract if it gets stuck. For instance, the elongated shaft is at least partly flexible to facilitate navigation through the gastrointestinal system. The elongated shaft may therefore have a tendency to follow the greater curvature of the stomach and may get stuck. The attachment element helps lift the elongated shaft away from the greater curvature by pulling on the flexible line.

In some embodiments the attachment element is configured, or is further configured, to assist the removal of an implant sheath from the gastrointestinal tract. To achieve this, the attachment element of the delivery system is configured to at least partly attach to any one or more of: an implant; an implant sheath; or an extraction mechanism of an implant sheath. Additionally or alternatively, the attachment element can be configured to at least partly encircle any one or more of: an implant; an implant sheath; or an extraction mechanism of an implant sheath.

The removal of an implant sheath is generally possible by pulling the attachment element via the flexible line. For instance, an extraction mechanism of the implant sheath may be operated such that the sheath releases the implant. Generally speaking, the extraction mechanism is only actuated once the implant is positioned at the target site. The attachment element may then be pulled to operate the extraction mechanism of the sheath and thus allow the removal of the sheath from the gastrointestinal tract. This may be possible for instance, by applying a greater pulling force on the attachment element as compared to the force applied when repositioning the implant.

The delivery system may be used with an auxiliary medical device. For example, the attachment element including a snare may hold a connection element of an implant or the sheath of an implant.

The connection element may be a connection element as described in the patent application with the international patent application No. PCT/EP2024/076814 filed on 25 September 2024 which is hereby included by reference. In the present invention, a similar or the same connection element of the international patent application may be used.

In some embodiments, the attachment element and flexible line are introduced within the gastrointestinal tract via a channel of the handle such that the flexible line extends at least partly parallel to the elongated main shaft of the delivery system. Alternatively, they may be introduced via a port of the handle. Furthermore, the snare, preferably the flexible line may be further adapted to translate within the channel of the handle and/or the elongate main shaft (e.g. by a pushing and/or pulling motion). The main shaft may comprise a guide for the flexible line. For example, when inserted though the elongated main shaft, the guide may be formed by an opening through which the line exits the main shaft. Additionally or alternatively, the guide may be another element such as a ring through which the flexible line passes and by which the flexible line is slidingly held.

Thereby excessive force on tissue, and associated irritation and injury, may be avoided.

A target site may be understood as being a predefined point or area within the gastrointestinal tract of a patient and where the implant is to be positioned and/or delivered.

The elongated main shaft of the delivery system is tube-like and may have a proximal end and a distal end. The elongated shaft may have at least one working channel extending at least substantially the length of the elongated shaft. Optionally, the working channel may extend from an opening at, or near, the proximal end to an opening at, or near, the distal end of the elongated shaft. Alternatively, the elongated shaft may have a plurality of working channels. The distal end of the elongated main shaft is coupled to a proximal end of the implant holder.

At least a portion of the elongated main shaft may be at least partly flexible such that the shaft may deform from and resume an initial shape without breaking. The elongated main shaft may thus be made of a flexible material. The material can comprise any one or more of: a silicone; a rubber; a polymer.

In some embodiments the elongated main shaft may comprise at least one reinforcement. Optionally, the elongated main shaft may comprise a plurality of reinforcements positioned along the length of the elongate main shaft. The reinforcement(s) may include a metal or a metal alloy.

The reinforcement(s) may be any one or more of: a spiral; a braid; a coil; a lattice; a strut; a mesh; a ring.

Generally speaking, the main shaft has a length which allows at least the positioning of the implant holder at, or near, the pylorus for the deployment of the implant. For instance, the elongated main shaft may have a length of at least 1000 mm-2000 mm. Preferably, the elongated main shaft has a length of at least 1300 mm. The elongated main shaft may have a diameter of between 16 mm-20 mm.

The medical device may, in particular, comprise a line attached to the attachment element.

In some embodiments, the medical device comprises an implant holder configured to hold an implant. The implant may be an intestinal sleeve. The implant holder may be coupled to, or is couplable to, the distal end of the elongated shaft. The implant holder may in particular be configured as an inner shaft arranged within and extending from the elongated main shaft.

The medical device may thus be configured as a delivery device adapted for insertion within the gastrointestinal tract of a patient. The device may be dimensioned such that it can be navigated through the gastrointestinal tract to the duodenum of the patient. Preferably, the delivery system is dimensioned such that the implant holder can be positioned proximal to the pylorus of the patient and the detachable distal tip positioned distal to the pylorus for the delivery of the implant.

The implant holder may have a hollow interior defined by an inner surface of the implant holder. The implant holder may thus comprise a lumen. The implant holder may comprise an opening at a first end and an opening at a second end. The lumen of the implant holder may extend between the first and second opening of the implant holder. The implant holder may have a periphery at a first end and a periphery at a second end which are different from one another.

The implant holder may be an auxiliary medical device. The auxiliary medical device may have at least one wing member, a support member, a release mechanism. The release mechanism may be a release line. The at least one wing member and the support member are releasably connected or connectable by the release line such as to form a substantially loop-shaped cross-section. The release mechanism, preferably the release line, is arranged such that the connection between the at least one wing member and the support member is released when the release mechanism is activated, in particular when the release line is pulled.

The auxiliary medical device may be auxiliary medical device as described in the patent application with the international patent application No. PCT/EP2024/076814 filed on 25 September 2024 which is hereby included by reference. In the present invention, a similar or the same auxiliary medical device of the international patent application may be used.

Preferably the implant holder may be conical and thus its first end may be at least partly smaller than the second end. In this case the lumen of the implant holder gradually increases as it extends from the first end to the second end.

The implant holder may be configured to couple to the distal end of the elongated shaft. Preferably, the proximal end of the implant holder couples to the distal end of the elongated shaft. Alternatively, the implant holder may be arranged within the main shaft and extend therefrom at a distal end.

The implant holder may couple to the distal end of the elongated shaft by a joiner element. Optionally, the joiner element of the implant holder may interact with a complementary joiner element provided at the distal end of the elongated shaft. The joiner element may be any one or more of: an adhesive; a lock element (e.g. a luer lock); heat sealing; compression fitting; a snap-fit connection; a threaded connection; a clamp element.

Additionally or alternatively, the implant holder may couple the elongated shaft by a friction fit wherein the implant holder is held on the distal end of the elongated shaft by a friction force between the elongated shaft and the implant holder. This may be achieved by providing an implant holder with an opening at the proximal end which has a smaller diameter than the diameter of the elongate shaft at its distal end.

Alternatively, the implant holder may be integrally formed with the elongated shaft. In such case no joiner element is provided.

In some embodiments the implant holder is configured to couple to at least a first extremity of an implant. Optionally, the implant holder may be configured to couple to an implant anchor.

The delivery system may further comprise a working channel. The working channel may extend at least substantially the length of the elongated main shaft. The working channel is preferably configured to house at least a deployer for deploying a sleeve within the patient. The deployer may preferably be an intestinal sleeve deployer for deploying an intestinal sleeve within the patient.

The working channel of the delivery system may extend at least substantially the length of the main shaft. The working channel may be adapted to receive at least one tool. Said tool may translate along at least a portion of the working channel.

In some embodiments the working channel further extends at least substantially the length of the implant holder.

Substantially the length of the implant holder may be understood in context as being the extension of the working channel along at least a portion of the length of the implant holder. Preferably the working channel extends the length of the implant holder.

Thus, the implant holder may have a lumen. The implant holder lumen may be configured to allow the passage of any one or more of: a deployer, a guidewire or a tool in said lumen.

In some embodiments, an implant is attached and compressed by a jacket. The attachment element may then be arranged on the jacket. Preferably, the implant holder is positioned at a distal end of the elongated main shaft.

The jacket may be fixedly attached to the implant, so that positioning and steering of the main shaft is possible by pulling on the jacket until and unless the jacket is released. Thus, the line and/or the attachment element may serve a double function for steering/orienting and removal of the jacket.

The jacket may be releasable. The line may thus be adapted to remove the jacket when released.

In some embodiments, the delivery system also comprises an atraumatic tip which is preferably arranged at a distal end of the implant holder and/or of the delivery device and/or a separate element and which may be detachable. Alternatively, the atraumatic tip may be arranged distally of the implant holder and/or of the delivery device and/or a separate element. Preferably the atraumatic tip is detachable from the delivery system.

The atraumatic tip may be directly or indirectly attached to the medical device. For instance, the atraumatic tip may be couplable to a distal end of the deployer or tool which is inserted through the delivery system.

The atraumatic tip is configured to reduce the risk of injury to the patient during the insertion and navigation of the delivery system through the gastrointestinal tract for the delivery of an implant. The tip has an at least partly rounded profile to avoid damaging the gastrointestinal tract.

"Atraumatic" refers to structures which are not damaging and/or do not deform the patient anatomy during the delivery procedure of the implant. For example, spherical shapes such as balls may have a reduced risk of perforating the bowels upon delivery of the intestinal sleeve.

The tip may in some embodiments be spherical; bulb-like; tapered. In a preferred embodiment, the atraumatic tip is a sphere.

It is conceivable that the atraumatic tip may have a lumen extending through the length of the tip. When attached to the delivery system, the lumen of the tip may align with the working channel of the delivery system. Additionally or alternatively, the lumen of the tip may align with the lumen of the deployer. Providing the tip with a lumen allows for a guidewire to be positioned within the gastrointestinal tract for guiding the insertion of the delivery system.

The atraumatic tip may be an atraumatic tip as described in the patent application with the international patent application No. PCT/IB2023/000161 filed on 10 March 2023 and published with the publication No. WO2023170478A1, which is included herein by reference. In the present invention, a similar or the same atraumatic tip of the International patent application may be used.

The invention is further directed to a ... elongated main shaft comprises a handle arranged at, or near, a proximal end of the shaft. The handle has a main body to allow for the gripping of the delivery system. This may help stabilise the delivery system during the delivery of the implant within the gastrointestinal tract.

Preferably, the handle comprises a detachable portion. The detachable portion of the handle may be at least one extremity of the handle. For instance, the first portion of the handle may be configured to detach from the second portion which remains attached to the delivery system.

It is conceivable that a substantial portion of the handle is detachable. In this example, the first portion may detach from a distal portion of the handle which remains attached to the delivery system.

The handle may comprise at least one channel extending from an opening at the proximal end of the handle to a distal end coupled to the proximal end of elongated shaft. It is conceivable that the handle may comprise a plurality of channels. At least a first channel of the handle is in communication with the working channel of the elongated shaft. The handle may comprise at least a second channel. The second channel can be arranged adjacent to the first channel. Preferably, the second channel runs at least substantially parallel to the first channel. The second channel may house any one or more of a flexible line; an inflation line; a release line. The handle may comprise an opening at its distal end through which the line housed within the second channel exits the handle. Additionally or alternatively, the second channel may connect with the channel of the elongate shaft and a second channel of the implant holder. In this case, the implant holder can comprise a first and second opening at its distal end. The fist opening of the implant holder allows the extension of the deployer beyond the implant holder. The second channel of the implant holder allows the exit of the line housed within the second channel of the implant holder.

The proximal end of the elongated main shaft may preferably carry the handle for handling the delivery system. This may facilitate the handling and/or the actuating of the delivery system by the operator.

In some preferred embodiments, the handle may be detachable from the elongated shaft of the delivery system. This may facilitate the removal of a tool or of a deployer from within the elongated main shaft of the delivery device.

In some more preferred embodiments, a portion of the handle is detachable..In this case the detachable portion detaches from the handle body. The detachable portion of the handle is configured to remove a deployer, and/or a support arranged in the main shaft, and/or a tool of the delivery system. To achieve this, the detachable portion may be positioned at a first extremity of the handle body, preferably the proximal most extremity of the handle. The detachment of the first extremity may cause the translation (e.g. by a pulling motion) of the deployer, or any other part(s) of the delivery device arranged in the main shaft, or a tool along the working channel of the elongated main shaft. The translation of the deployer may be bidirectional and thus the detachable portion may further serve to push the deployer or a tool along the working channel.

Whilst the detachable portion is detached from the delivery system, the handle body remains attached to the elongated main shaft of the delivery system which in turn holds the implant at the target site, for example via the implant holder. This may facilitate the maintaining the implant in a target position whilst enabling a safe and effective removal of the deployer (via the detachable portion) with a reduced risk of disturbing the position of the implant.

Detaching the detachable portion of the handle may facilitate the removal of a tool or of a deployer from within the elongated main shaft of the delivery device. Removing the deployer and/or tool whilst the delivery device remains in the position may help reduce the duration of the intervention as it streamlines the process on inserting and removing various instruments during the intervention. Additionally, removing the detachable portion of the handle can help facilitate the positioning of the implant at the target site.

The handle and the detachable portion of the handle may comprise a locking system to secure the portions of the handle together. The locking system may comprise at least one lock component. Preferably both the detachable portion of the handle and the handle main body each have a lock component configured to cooperate with one another for securing the detachable portion to the main body of the handle. Thus, the detachable portion of the handle may comprise a lock component which interacts with the lock component of the main body of the handle.

The detachable portion may connect to the handle body by any one or more of: a threaded connection; a snap-fit connection; press-fit connections; a clamp; a latch; a clip; a pin; a magnet; a rotary lock; a slide lock; a bayonet lock; a pin or bi-pin twist lock; a Storz lock or Storz-type lock.

The delivery system may, in some preferred embodiments, further comprise a deployer. The deployer is configured to translate at least substantially the length of the working channel for deploying the implant.

The deployer may extend beyond the distal end of the elongated shaft and through the implant holder.

In a preferred embodiment the deployer can be introduced into the working channel at the proximal end of the elongated shaft. The deployer then translates the length of the elongated shaft to deploy the implant. Furthermore, the deployer may be configured to extend beyond the working channel such as to extend at least a portion of the implant, preferably an intestinal sleeve of the implant, from an insertion configuration (i.e. at least partly longitudinally compressed) to a deployed configuration (i.e. at least partly longitudinally extended).

It is conceivable that the deployer may be introduced into the working channel of the elongated shaft of the delivery system somewhere along the elongated shaft, for example via a port of the delivery system which is distanced from the proximal end of the elongated shaft. In this case the deployer translates part of the length of the working channel for the longitudinal extension of the implant.

To achieve this, the deployer may be attached, or attachable, to a distal end of the implant. Additionally or alternatively, it is conceivable that the deployer may be attached, or attachable, to the detachable distal tip of the delivery system which may in this case be attached to the implant, preferably the distal end of the implant.

The deployer may, in some embodiments, extend through the elongated main shaft in the working channel, through the implant holder via the implant lumen and connect to the atraumatic tip of the delivery system.

Translation of the deployer may push the implant and/or the tip thus inducing the extension of the implant from the longitudinally compressed state to the longitudinally extended state.

It is conceivable that the deployer may comprise a lumen extending along its length. The lumen of the deployer may at least partly align with a portion of the working channel of the elongated main shaft. Such that a tool inserted through the working channel may also be passed though the deployer. For example, the deployer lumen may allow the passage of a guidewire along the delivery system, which is inserted through the working channel. The guidewire may be provided for guiding the insertion of the delivery system.

In some embodiments the working channel may house, or may further houses, a guide wire for guiding the delivery system.

If, and when, the guidewire is provided, the deployer is arranged concentrically around the guidewire.

In turn the elongated shaft of the delivery system is arranged concentrically around at least a portion of the deployer. In some cases, the deployer may extend beyond the elongated shaft and therefore the elongated shaft is arranged concentrically around the portion of the deployer which is within working channel.

The attachment element may be any one or more of: loop-like, lasso-like, noose-like.

In some embodiments the delivery system comprises or further comprises at least one port. The at least one port may be arranged at or near the handle. Optionally, the at least one port may be in communication with the working channel of the delivery system.

Generally speaking, the port comprises an opening and may connect to a channel through which at least one tool and/or fluid can be introduced. The channel extends from the opening and runs along the length of the elongated shaft to the distal end. The channel guides and directs the instruments, fluids, or gases to the specific area being examined or treated.

The port may allow the introduction of various tools the operator may require during the intervention. For instance, the port may serve to introduce a light source to illuminate the gastro-intestinal tract.

The tool may be any one or more of: an insufflation device; an inflation device; a suction device; an irrigation device; an imaging device.

The port may allow the introduction of various fluids the operator may require during the intervention. For instance, an irrigation fluid may be introduced within the gastrointestinal tract via the port.

The fluid may be any one or more of: an irrigation fluid or an inflation fluid. The port may further serve to remove a fluid from the gastrointestinal tract. For example, gastric content may be aspirated via the port.

Providing one or more ports may allow for a more compact device that is easily adaptable according to the needs of the operator and may allow said operator to easily adapt the procedure according to the specific needs of patients on a case by case basis or to easily respond to potential complications arising during the intervention.

When, and if, a port is provided, the at least one port may extend at least partly the length of the elongated main shaft. Additionally or alternatively the at least one port can be connected with the implant holder.

It is also conceivable that a plurality of ports may be provided.

At least one port may be any one or more of an insufflation port; an inflation port; an aspiration port; an optical port; an injection port; a light port.

When, and if provided, the insufflation port may be configured to receive an insufflation device for delivery of an insufflation fluid within the gastrointestinal tract.

The delivery of an insufflation fluid causes the gastrointestinal tract to move away from surrounding tissue as well as increasing the interventional area. This may help improve the operator's visualization of the procedure and may further provide for easier navigation and a larger area for operating the delivery system.

The inflation port may be configured to receive an inflation device for delivery of a fluid to the implant holder. Optionally, the inflation device delivers a fluid to the implant.

For example, the inflation device may supply a fluid to an inflatable implant anchor, such as a balloon anchor, for inflating the anchor to an anchoring configuration which anchors the implant within the gastrointestinal tract of the patient. In this example, the inflation port has an inflation channel configured to carry a fluid to the inflatable anchor via the implant holder. The implant holder may comprise a fluid tight connection at a coupling site between the implant holder and the inflatable implant anchor. The fluid tight connection may be provided by the coupling element. The fluid tight connection may comprise at least one valve. The valve may be provided on the implant holder and/or the inflatable anchor. The valve may be configured to allow unidirectional passage of a fluid from the inflation device to a chamber of the anchor.

The aspiration port is generally configured to receive a suction device for aspiration of a content of the gastrointestinal tract. The content of the gastrointestinal tract may relate to a fluid contained within the gastrointestinal tract. The fluid may be a liquid and/or a gas. Preferably, the fluid may be any one or more of: blood; irrigation fluid; gastric fluids; intestinal fluids; air. The content of the gastrointestinal tract may also be a solid contained within the gastrointestinal tract. The solid may be one or more of cellular debris; food.

The optical port is adapted to allow the introduction of an imaging device within the gastrointestinal tract for visualization of the gastrointestinal tract. The imaging device may be a camera; an ultrasound probe; a fiberoptic imaging device. The imaging device allow the operator to visualize the gastrointestinal tract from within whilst the delivery system is being operated.

The injection port is configured to house an injection device for the introduction of a fluid within the gastrointestinal tract. For example, a liquid solution may be delivered to the gastrointestinal tract by the injection device which is introduced into the tract via the injection port. The injected fluid may serve to irrigate the gastrointestinal tract during the intervention.

A light port is configured for introducing a light source within the gastrointestinal tract for illuminating the gastrointestinal tract. The light source serves to illuminate the gastrointestinal tract and improve the visualization of the tract during the intervention.

In some embodiments, an imaging device may be introduced into the gastrointestinal tract independently of the delivery system.

In such a case the imaging device can be introduced in parallel to the delivery system. This advantageously allows for uninterrupted visualization of the gastrointestinal tract during the positioning of the delivery system and during the delivery of the implant by the delivery system. The continuous visualization may improve the chance of success of the delivery of the implant as any rectification required to the position of the implant or rectification of the position of the delivery system for successful delivery or anchoring of the implant can be seen immediately and the necessary adjustment made.

In another aspect, the invention is directed to a medical device, e.g. a delivery device and/or and endoscope which comprises an elongated main shaft with a handle arranged at, or near, a proximal end of the shaft. The handle may be at least partly detachable, preferably wherein the handle (50) comprises a detachable portion. The medical device may be a device as described above, and/or may have any of the aspects described.in context with a first aspect of the disclosed invention.

It is conceivable that certain elements of the medical device are attached to a first part of the handle, which is not detachable, whereas other parts are attached to the detachable part. Thereby, it can be possible to selectively remove certain parts of the medical device after use without having to remove other parts of the medical device. These removable parts can still be fixedly attached to the medical device (i.e. the detachable handle portion) and thus provide increased safety.

The medical device may further comprise a deployer, said deployer being configured to translate at least substantially the length of a working channel for deploying an implant.

The deployer may be attached to the detachable portion of the handle. The deployer may thus be removed through the working channel by removal of the detachable handle.

Thus, it may be possible to deploy an implant and, subsequently, remove parts of the medical device which are used for implant deployment and no longer needed. The main shaft may, however, may remain in the patient for further procedures, for example introduction of an endoscope and/or instruments for sewing.

In some embodiments the atraumatic tip of the delivery system is coupled to a distal end of the deployer by a releasable connector. The releasable connector allows the detachment of the atraumatic tip from the deployer.

The releasable connector may be any suitable means which is configured to realisably attach the distal tip to the delivery system. For instance, the distal tip may connect to the delivery system by means of any one or more of a clip; jaws; a gripper; a magnetic connection; a phase transition element.

In some preferred embodiments the releasable connector may comprise a first connector part and second connector part. The first connector part and the second connector part may comprise one or more of: a magnet, a gripping unit, a phase transition component, a wire.

It is conceivable that in a preferred embodiment the releasable connector is an expandable and/or contractable gripper. The connection may be released by expanding and/or contracting the gripper. In one form, the gripper can change configuration between an expanded configuration and a contracted configuration. In one configuration (e.g. the expanded configuration), the gripper is configured to couple the deployer to the atraumatic tip component, for example, by expanding within a socket of the tip component to grip the socket from within. In the other configuration (e.g. the contracted configuration), the gripper is configured to release the atraumatic tip, for example, by disengaging from within the socket of the tip component.

Another example of releasable connector may be a magnetic connection between first and second connector parts. The magnetic connection may be formed by at least one permanent magnetic, optionally first and second permanent magnets. The connection may be releasable by manipulating the deployer to force apart the first and second connector parts. For example, one connector part may be retracted into a lumen of the deployer to separate it from the opposite connector part in the atraumatic tip. In another example, an electromagnetic coupling may be used. The coupling may be released by reducing or removing application of an electric current to an electromagnetic connector part.

The releasable connector may comprise a phase transition element having two distinct phases. For example, the phase transition element may comprise or consist of expandable elements of having an austenite and a martensite phase. The phase transition element may be adapted to provide a fixed connection between the deployer and the atraumatic ball in a first phase (e.g. in the austenite phase), and release the atraumatic tip component from the deployer in a second phase (e.g. in the martensite phase). The martensite phase in phase transition materials is generally more mechanically flexible than the austenite phase. Therefore, an expandable element may provide a fixed connection in austenite phase, wherein said connection may be released by being pulled out in the martensite phase in which it may be easily deformable.

In some embodiments the releasable connector may be a releasable connector as described in the patent application with the International patent application No. PCT/IB2023/000161 filed on 10 March 2023. In the present invention, a similar or the same releasable connector of the International patent application may be used. The releasable connector is hereby included by reference.

The atraumatic tip is preferably configured to retain the implant in an insertion configuration.

This may be achieved by the pinching or holding of the distal end of the implant, preferably a sleeve implant, between the distal tip of the delivery system and deployer. The implant may be held or pinched by the releasable connector which attaches the distal tip to the delivery system. For instance, when the releasable connector is a gripper, the distal end of the implant may be held between the gripper and the socket of the tip.

In the insertion configuration the implant may be at least partly compressed radially. Additionally or alternatively, in the insertion configuration the implant may be at least partly compressed longitudinally.

Upon the release of the detachable atraumatic tip, the distal end of the implant is no longer held by the releasable connector. This allows for the implant to passively and/or actively assume a deployed configuration.

In the deployed configuration the implant may be fully expanded radially and/or fully extended longitudinally.

In some embodiments the atraumatic tip is configured to at least partially transition the implant from the insertion configuration to the released configuration upon detachment of the atraumatic tip.

The insertion configuration of the implant is understood as being a state in which the implant is at least partly compressed circumferentially compared to the circumference of the implant in a non-compressed state which is the deployed configuration.

Additionally or alternatively, in the insertion configuration the implant may be compressed longitudinally. In this case, the atraumatic tip prevents the implant from assuming its full longitudinal length. Once the tip is released from the delivery system the implant may·be passively and/or actively extended longitudinally.

In the released configuration the implant may be at least partly expanded and/or extended compared to the insertion configuration but may not be fully expanded and/or extended as in the deployed configuration.

For instance, the delivery system may be provided such that a circumferentially compressed implant is provided between the implant holder and the detachable tip. The implant may further be longitudinally compressed. The detachable tip may be attached to the delivery system by a releasable connector provided at the distal extremity of the implant holder such as a grasping element which grasps the detachable tip and a portion of the implant may be grasped by the grasping element such that it is positioned between the grasping element and the distal tip. The deployer may be translated to transition the implant to a longitudinally extended configuration. It is maintained in the circumferentially compressed configuration by the distal tip and the releasable connector of the deployer. The releasable connector may be actuated such that the detachable tip disconnects from the deployer thus releasing the implant grasped between the detachable tip and the deployer. Upon the release of the detachable tip from the device the implant may transition from the circumferentially compressed configuration given to the implant for delivery of the implant within the patient to the circumferentially released configuration. The implant may then be further expanded circumferentially and/or further extended longitudinally to reach the fully deployed configuration.

The further extension and/or expansion of the implant may be achieved passively and/or actively. In a first example, a passive circumferential expansion of the implant may be achieved by injecting a fluid through the implant. For this a liquid may be injected into the lumen of the bypass sleeve. The force of the fluid flow though the sleeve induces the circumferential expansion of the sleeve.

In a second example, an active expansion of the implant may be achieved using an inflation device to inflate an inflatable implant anchor.
attachment elementattachment elementattachment elementattachment element
Thus, in some preferred embodiments, the delivery system comprises an imaging device. Alternatively, in some more preferred embodiments the imaging device is provided in parallel with the delivery system.

The imaging device can be deflectable with regards to a longitudinal axis of the elongated main shaft. The imaging device may be deflected from the main shaft to provide a better visualisation during the navigation and delivery of the implant to the gastrointestinal tract.

As a first example, the imaging device may be inserted through an optic port of the handle. In this case, the imaging device is substantially parallel to the elongated main shaft and the imaging field is obstructed by the delivery system. By deflecting the imaging device away from the longitudinal axis of the elongated main shaft, the viewing field is changed and preferably, provides an improved visualisation of the field.

The deflection of the imaging device may be operated by an actuator of the delivery system.

In some preferred embodiments the imaging device may comprise the steerable core. This could allow the navigation of the imaging device within the gastrointestinal tract such that an unobstructed visualisation of the positioning of the delivery system and the delivery of the implant may be provided.

In some embodiments the elongated main shaft comprises a steerable core which extends at least a part of the length of the main shaft. This may be achieved by providing one or more articulations to the steerable core. The elongated shaft may be made of an at least partly flexible material which is able to deform under the steering of the core.

The steerable core may comprise any one or more of: control wires; articulated segments; a hydraulic mechanism; a pneumatic mechanism; a shape memory alloy.

In some preferred embodiments the delivery system comprises an actuator for changing the position of the imaging device, preferably by changing the position of the steerable core from a first position to a second position. Preferably, the actuator is provided at the handle of the delivery system.

Alternatively, if the imaging device is provided separately to the delivery system, the actuator may be provided at a proximal end of the imaging device.

It is conceivable that the actuator may be further configured to translate the deployer along the main shaft.

It is further conceivable that the actuator may actuate the releasable connector attaching the distal tip to the deployer.

In some embodiments the working channel is configured to house an anchor deployer for deployment of at least one implant anchor within the gastrointestinal tract.

In some embodiments the working channel may additionally or alternatively, be configured to house an extraction mechanism for removing an implant sheath from the implant and/or for retrieval of the implant sheath from within the gastrointestinal tract.

In some embodiments, at least one port is configured to house at least one of the anchor deployer or the extraction mechanism.

It is conceivable that the working channel may house one of the anchor deployer or the extraction mechanism whilst the port houses the other. For example, the working channel may house the anchor deployer and the port may house the extraction mechanism.

It is possible that one of the working channel or the port houses both the anchor deployer and the extraction mechanism. For instance, the working channel may house both the anchor deployer and the extraction mechanism. In this case the port may house a tool as discussed herein. The same example is possible wherein the port houses the anchor deployer and the extraction mechanism.

As a further example, when the deployment device comprises a plurality of ports, it is possible that the anchor deployer and the retrieval line be housed in different ports. The same applies when the delivery system comprises a plurality of working channels, the anchor deployer and the extraction mechanism may be provided in separate working channels.

In some embodiments the anchor deployer and the extraction mechanism may be provided as a one component combining the features of both individual components.

The anchor deployer may be an inflation device for the inflation of a balloon anchor.

The extraction mechanism may comprise a release line. The release line may be configured to open the sheath such that the implant is no longer constrained. The sheath may retain at least one implant anchor in a constricted configuration. Optionally the sheath may retain a gastric anchor and a duodenal anchor of the implant.

Thus, the extraction mechanism may be configured to passively deploy an anchor by removal of the sheath.

The extraction mechanism may further comprise a retrieval line. The retrieval line may be configured to allow removal of the open sheath from within the gastrointestinal tract.

In some embodiments, the extraction mechanism is introduced within the gastrointestinal tract via a channel of the handle such that it extends at least partly parallel to the elongated main shaft of the delivery system. Alternatively, the extraction mechanism may be introduced via a port of the handle.

In a preferred embodiment the anchor deployer comprises an inflation line configured for active deployment of the at least a one implant anchor. The inflation line may be configured to inflate the implant anchor. This is achieved by the delivery of a fluid to the anchor via the inflation line.

The inflation line may be an inflation tube for inflating at least one implant anchor, preferably a balloon anchor. The anchor deployer may connect to the implant anchor via the implant holder to allow inflation of the implant.

The delivery system may further comprise an implant connected to the implant holder. Optionally, the implant is a sleeve implant, preferably a bypass sleeve. The implant preferably couples to the implant holder via the coupling element of the implant holder.

The implant comprises a gastric anchor configured to anchor the implant in the stomach of the patient. The implant further comprises a duodenal anchor configured to anchor the implant in the duodenum of the patient.

The gastric anchor preferably couples to the implant holder of the delivery system. The gastric anchor may comprise a coupling element configured to couple with coupling element of the implant holder.

In some embodiments the implant, or any part of the implant, may be the implant, or any part of the implant, as described in the patent application with the international patent application No. PCT/EP2024/050497 filed on 10 January 2024 and which is incorporated herein by reference. In the present invention, a similar or the same implant of the International patent application may be used.

The implant is configured to transition from an insertion configuration to a released configuration. In the insertion configuration the implant may be at least partly compressed. Preferably the implant and optionally the implant anchors may be compressed circumferentially such that the implant has a reduced circumference compared to a non-compressed state. In the insertion configuration the implant may have a diameter of 15 mm to 22 mm, preferably 17 mm-20 mm.

In the insertion configuration at least one implant anchor may have a circumference of 15 mm to 22 mm. Preferably, the implant anchor has a circumference of 17 mm to 20 mm in the insertion configuration.

In some embodiments, in the insertion configuration, at least one anchor of the implant may have a circumference which varies along the length of the anchor. In other words, at least one implant anchor may have different circumferences along its length.

In the insertion configuration of the implant, the sleeve of the implant, such as an intestinal sleeve, has a reduced circumference compared to the circumference of an anchor of the implant in the insertion configuration.

Additionally or alternatively, in the insertion configuration the implant may be at least partly compressed longitudinally. In this case the implant is compressed such that its length is less than the length of the implant in a non-compressed state. The non-compressed state may be the deployed configuration of the implant.

For instance, the length of the implant in a non-compressed state may be between 600mm-1200mm. Preferably, in the non-compressed state, the length of the implant is between 700 mm to 1000 mm. Even more preferably, the implant has a length of 715 mm in the non-compressed sate. In the longitudinally compressed state, the implant sleeve, preferably an intestinal sleeve, may have a length of 100 mm to 400 mm, more preferably 150 mm to 250 mm.

It is conceivable that the implant is configured to transition from the insertion configuration to a released configuration and may further transition to a fully deployed configuration.

In a further aspect, the invention relates to a method of delivering an implant. The method optionally uses the medical device according as described herein above. Optionally the method may use the implant system according to the third aspect of the invention. The method comprises any one or more of the following steps, in any order:
- providing an implant, optionally a gastro-intestinal sleeve, preferably a gastrointestinal sleeve comprising an anchor, to the implant holder,
- inserting the delivery system within the gastrointestinal tract of a patient, preferably within the duodenum;
- advancing the implant distally via a deployer until the implant, preferably the sleeve, is longitudinally deployed;
- releasing the atraumatic tip from the delivery device, preferably by actuating a releasable connector of the deployer
- transitioning the implant, preferably a sleeve, from an circumferentially compressed configuration to a circumferentially deployed configuration, optionally by injection of a fluid along the sleeve,
- actuating an anchor deployer for deploying an implant anchor of the implant,
- steering a distal portion of the delivery device by pulling on a line which is attached to said distal portion via an attachment element, and/or releasing the implant from a jacket and removing the jacket by pulling on the line.

In some embodiments the method further comprises a step of adjusting the position of the implant using a snare of the delivery system. Preferably the position is adjusted by the pushing and/or pulling of the snare.

In some embodiments the method may further comprise at least one of the following steps:
- actuating the anchor deployer for passively deploying a first implant anchor,
- actuating the anchor deployer for actively deploying a second implant anchor,
- operating a snare, preferably the flexible line of the snare, for positioning an implant relative to a target site. The snare, preferably the flexible line, is preferably operated by a translational motion such as pulling and/or pushing.
- using an extraction mechanism and/or a snare, to retrieve an implant sheath. The retrieval of the implant sheath is preferably achieved by pulling the extraction mechanism and/or snare.

In a third aspect the invention relates to an implant delivery assembly for delivery of an implant within the gastrointestinal tract. The implant delivery assembly comprises a delivery system according to the first aspect of the invention. The implant delivery assembly further comprises a gastrointestinal implant, optionally a duodenal implant. The implant has a tubular body extending from a proximal end to a distal end. The proximal end of the implant comprises at least one implant anchor. The at least one implant anchor is expandable from a non-expanded configuration to an expanded configuration. The distal end of the implant is coupled to the atraumatic tip of the delivery system.

In a preferred manner, the implant is a gastrointestinal sleeve, even more preferably a duodenal sleeve. The proximal end of the implant is adapted to attach to the implant holder of the delivery system. The implant may attach to the implant holder via the at least one anchor at the proximal end of the implant. To achieve this the implant anchor may comprise a coupling element configured to couple with a coupling element of the implant holder. The implant holder may serve to expand the implant anchor from the non-expanded configuration to the expanded configuration.

In the expanded configuration the implant anchor is adapted to retain the implant within the gastrointestinal tract. Optionally, the at least one implant anchor is actively expandable. For instance, the at least one anchor may transition from a non-expanded configuration to an expanded configuration by introduction of a fluid within the anchor. This is achieved by providing the at least one anchor with a chamber configured to hold the fluid.

Alternatively, the at least one anchor may passively expand due to the properties of the materials it is made from. For example, the anchor may be made of a material which resumes its original shape once no longer retained in a non-expanded configuration. The material may be a memory shape material, an elastic material. The material may include a polymer such as silicone.

The distal end of the implant is coupled to the detachable tip of the delivery system preferably by means of the releasable connector of the deployer of the delivery system.

In a preferred embodiment, the implant is provided in a compressed state and attaches to the implant holder. The deployer extends through the working channel of the delivery system; through the lumen of the implant holder; and within the tubular body of the implant. The implant holder is adapted to grasp the distal end of the implant and connect to the detachable tip via the releasable connector.

In a preferred embodiment the implant comprises a sleeve coupled to the implant anchor. Preferably the sleeve is coupled to a duodenal anchor. The duodenal anchor may be configured to anchor the sleeve within the duodenum of a patient.

In some embodiments the duodenal anchor may be the duodenal anchor as described in the patent application with the International patent application No. PCT/EP2024/050497 filed on 10 January 2024. In the present invention, a similar or the same duodenal anchor of the International patent application may be used. The duodenal anchor is hereby included by reference.

In some preferred embodiments the implant comprises a sheath. The sheath is configured to retain at least one implant anchor in a non-expanded configuration.

The sheath may be configured to hold two anchors in the non-expanded configuration.

In the non-expanded configuration, the anchor is at least partly compressed to a smaller size compared to the expanded configuration. Preferably, in the non-expanded configuration the anchor is at least circumferentially compressed. Additionally or alternatively the anchor may be longitudinally compressed.

In a preferred embodiment at least part of the implant sheath attaches to the snare of the delivery system. Additionally or alternatively at least part of the sheath is encircled by the snare.

The sheath comprises an extraction mechanism for deployment of at least one implant anchor from a non-expanded configuration to an expanded configuration. The extraction mechanism comprises a release line configured to release an implant sheath. The release of the sheath may passively deploy the at least one implant anchor from a non-expanded configuration to an expanded configuration. The release line may be configured to open the sheath. This may remove the sheath from around the at least one implant anchor. Preferably, the release line may remove the sheath by a pulling force. The pulling of the release line may cause the sheath to tear thus releasing the at least one anchor which is held in a non-expanded configuration by the sheath.

Alternatively, the release line may be provided as a sew line configured to retain the sheath in a closed configuration. The sew line may have a chain stich profile or single pull stich profile. Pulling of the release line causes the stitches of the sew line to undo, thus opening the sheath.

The release line may provide for an easier removal of the sheath from the implant such as to facilitate the deployment of the implant anchors.

When, and if, a plurality of anchors are provided, the sheath may hold the plurality of anchors in the non-expanded configuration. Furthermore, it is possible that the sheath holds at least a portion of the implant sleeve. For example, the implant sleeve may be provided in a circumferentially and/or longitudinally compressed state. The compressed implant may be housed within the sheath and be released by the opening of the sheath.

Additionally or alternatively, the extraction mechanism comprises a retrieval line configured to remove the implant sheath from within the gastrointestinal tract. The retrieval line may be operated by a pulling force.

Generally speaking, the retrieval line is adapted to retrieve the sheath from the gastrointestinal tract of the patient after the anchor has been released from the non-expanded configuration. The retrieval line may be attached to the sheath by an adhesive. Alternatively, the retrieval line may be embedded within the sheath. Further alternatively, the retrieval line may be sewn onto the sheath.

The retrieval line may attach to the anchor deployer of the delivery system. Additionally or alternatively, the anchor deployer may be configured to pull the retrieval line for the retrieval of the sheath.

The extraction mechanism may attach to the snare of the delivery device. In this case, the extraction mechanism may be operated by the snare (e.g. by pulling the snare). Thus, the release line and/or the retrieval mechanism of the extraction mechanism may attach to the snare of the delivery device.

In some embodiments the retrieval line and the release line may be combined into one element. Thus, the retrieval line may be configured to open the sheath. The retrieval line may therefore serve to release the anchor from the sheath.

In a preferred embodiment the implant comprises a first anchor connected to a second anchor. The first anchor is optionally a duodenal anchor, and the second anchor is optionally a gastric anchor, preferably a gastric balloon. At least one of the first or the second anchor is passively or actively deployable from a non-expanded configuration to an expanded configuration.

The duodenal anchor may be connected to a gastric anchor by a connector. The gastric anchor, the connector and the duodenal anchor may be configured to anchor the implant within the gastrointestinal tract of the patient.

In some embodiments the connector may be the connector as described in the patent application with the International patent application No. PCT/EP2024/050497 filed on 10 January 2024. In the present invention, a similar or the same connector of the International patent application may be used. The connector is hereby included by reference.

At least one of the anchors may be deployed by actively expandable via inflation. At least one anchor may be passively expandable due to the mechanical properties of the material it is made from. For instance, one anchor may be expandable by inflation with a fluid whilst the other may be expandable due to the elastic properties of the material it comprises (e.g. memory shape polymer).

The invention is now described with reference to certain embodiments and figures which show:
- Fig. 1:: a schematic representation of a first embodiment of a delivery system.
- Fig. 2: a schematic representation of a second embodiment of a delivery system.
- Fig. 3: a schematic longitudinal view of an embodiment of the implant of an implant delivery assembly.
- Fig. 4: a schematic longitudinal view of an alternative embodiment of the implant of an implant delivery assembly.
- Fig. 5a-b: a schematic representation of an embodiment of the handle of the delivery system in an attached and detached state.
- Fig. 6a-b: a schematic longitudinal section view of the handle of figs. 5a-5b.
- Fig. 7: a schematic representation of a method of delivering an implant with the delivery system of the first aspect of the invention.
- Fig. 8: a schematic representation of an embodiment of the delivery device and auxiliary medical device.
- Fig. 9a-9c: a schematic longitudinal section view of the delivery device of fig. 8.
- Fig. 10: a schematic longitudinal section view of the handle and implant holder of the delivery device.
- Fig. 11: a first schematic longitudinal section view of the handle and implant holder of the delivery device connected to an auxiliary medical device.
- Fig. 12: a second schematic longitudinal section view of the handle and implant holder of the delivery device connected to an auxiliary medical device in a different plane compared to Fig. 11.

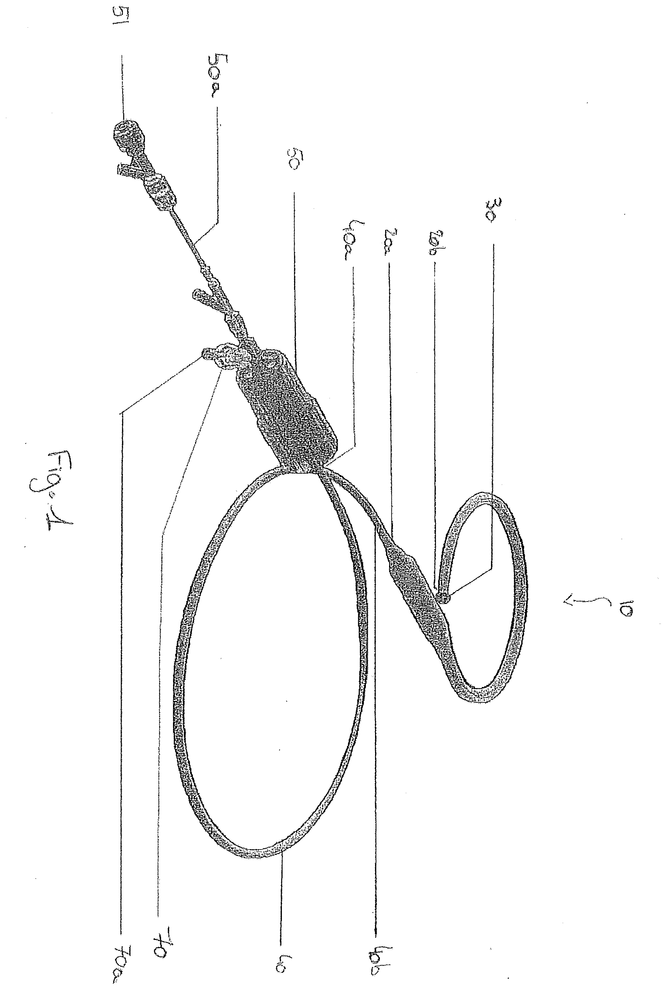

As seen in fig. 1 the delivery system comprises an implant holder 20 which is configured to hold an implant (not shown). The implant holder 20 is arranged at a distal end 40b of an elongated shaft 40 of the delivery system 10. A handle 50 is arranged at a proximal end 40a of the elongated main shaft 40.

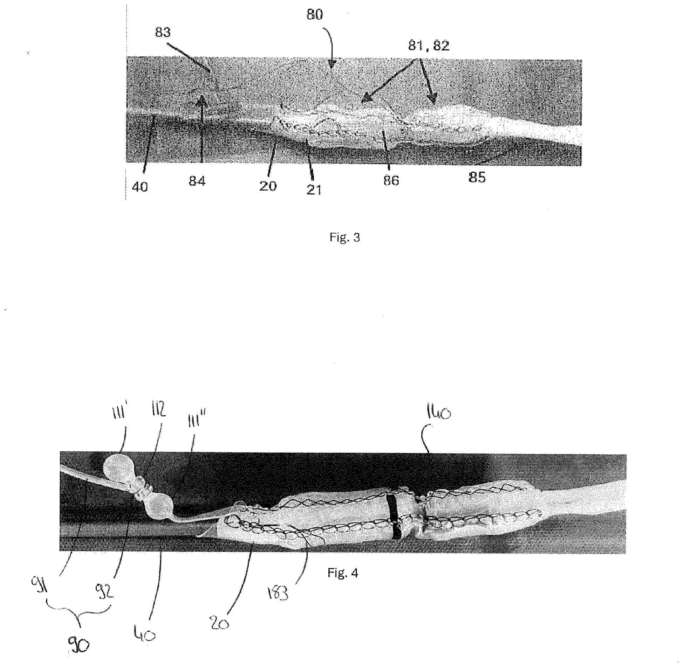

The handle comprises a channel 50a extending from an opening 51 at the proximal end of the handle toward the proximal end 40a of the elongated shaft 40. The handle channel at least partly aligns with of the working channel (see fig. 4) of the main shaft. The handle 50 further comprise a port 70. The port 70 comprises an opening 70a and a channel (not shown). The port channel may connect to the handle channel and/or the working channel of the elongated shaft 40. Said port 70 may be used to introduce a tool (not shown) within the elongated shaft 40 to facilitate the delivery of the implant. The handle 50 is coupled to the elongated main shaft 40.

The distal end 40b of the main shaft 40 is coupled to the proximal end 20a of the implant holder 20. The proximal end 20a of the implant holder 20 is adapted to attach to an implant via a coupling element (not shown).

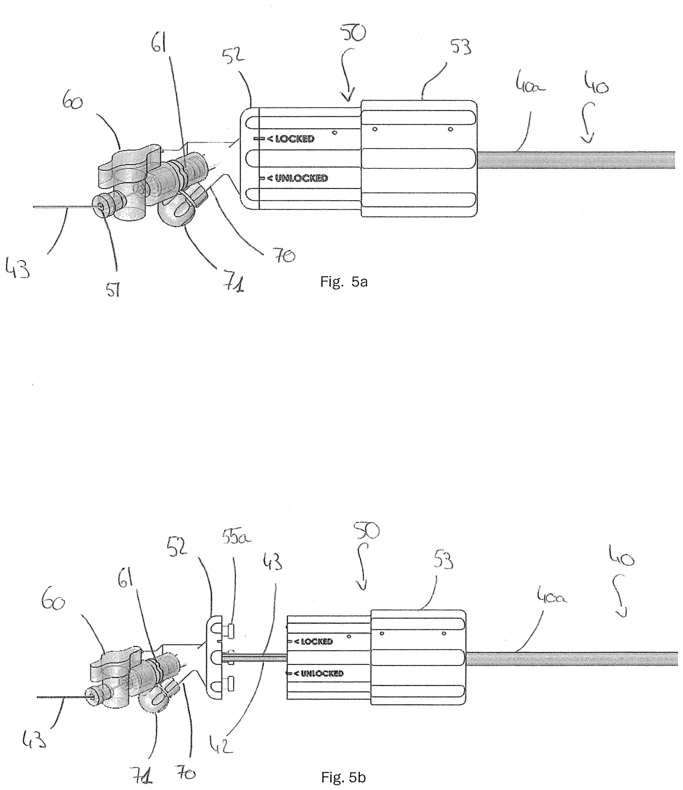

The delivery system 10 has a detachable atraumatic tip 30 arranged distally 20b of the implant holder 20. The atraumatic tip 30 attaches to a deployer 42 (see fig. 5a-b) via a connector (not shown). The deployer 42 extends within the working channel 41 of the delivery system 10 and is configured to translate therewithin.

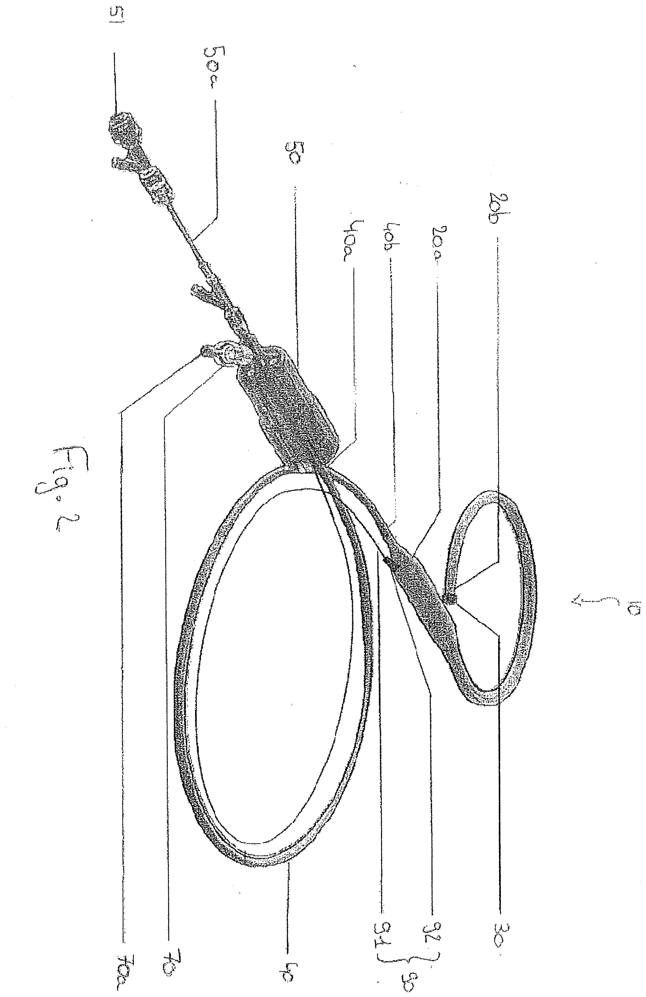

Shown in fig. 2 is a similar delivery system to one shown in fig.1. Similar elements as in Fig. 1 are not described again for clarity. Here, the delivery system further comprises an attachment element 90, preferably a snare. The snare 90 is configured to position the implant with regards to a target site and/or to remove an implant sheath (see fig. 3) from the gastrointestinal tract. The snare 90 comprises an attachment element 92 and a flexible line 91. The attachment element 92 attaches the line 91 to the implant sheath. The flexible line 91 is adapted to extend through a channel 50a or port 70 of the handle and to extend out of an opening of the handle 51 or an opening 70a of the port such that it the flexible line 91 may be pulled or pushed by an operator to position the implant and/or to remove the implant sheath. In some cases, the snare 90 attaches to the extraction mechanism of the implant sheath, for instance attaching to the release line 83 and/or the retrieval line 84 of the extraction mechanism (see fig. 3).

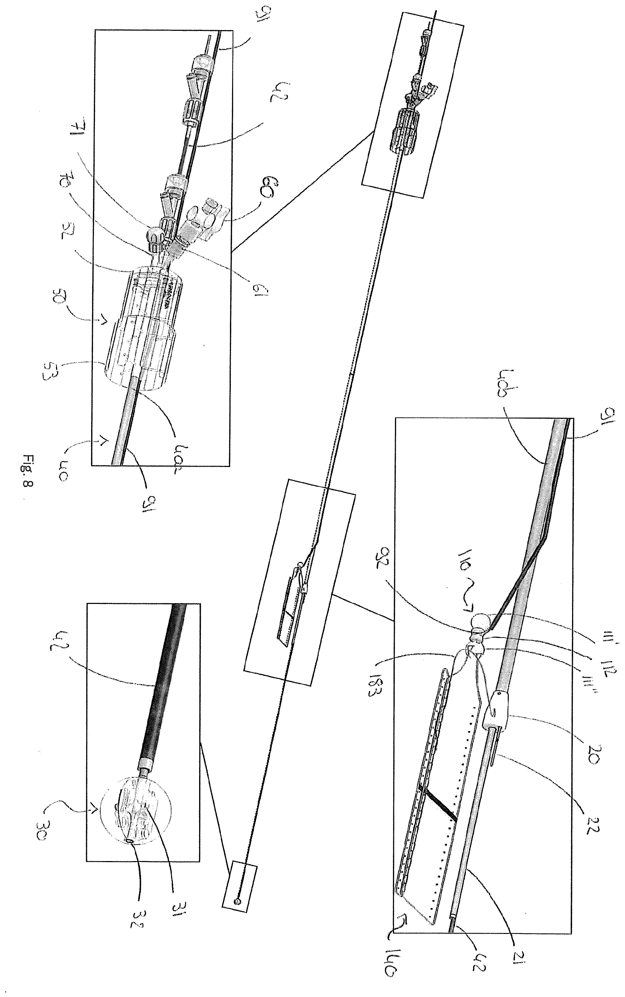

Fig. 3 shows a sleeve implant 80 of an implant delivery assembly and configured for delivery with a delivery system 10. The implant 80 is coupled to an implant holder 20 of the delivery system. The implant has two anchors 81, 82 for anchoring the implant within the gastrointestinal tract of a patient. One of the anchors is a duodenal anchor 82 and the second anchor is a gastric anchor 81. The anchors 81, 82 are connected to one another by a connector (not shown) and the duodenal anchor 82 is attached to a bypass sleeve 85. To anchor the sleeve implant 80, the duodenal anchor 82 and the bypass sleeve 85 are delivered to the duodenum of a patient by the delivery system. The gastric anchor 81 is an inflatable anchor (e.g. a balloon) to be positioned within the stomach of the patient, preferably within the pylorus. The gastric anchor 81 is inflated by an inflation device (see Fig. 8) connected to the anchor via the coupling element (not shown). The gastric anchor 81 of the device prevents the sleeve implant 85 from migrating distally along the intestine. The duodenal anchor 82 prevents the sleeve implant 85 from migrating proximally along the stomach.

The implant is retained by a sheath 86. The sheath 86 retains the duodenal anchor 82 and the gastric anchor 81 in a non-expanded configuration (i.e. compressed). The sheath 86 comprises an extraction mechanism having a retrieval line 84 and a release line 83. The release line 83 is adapted to allow the sheath to open upon pulling of the release line 83 thus releasing the duodenal anchor 82 and the gastric anchor 81. The retrieval line 84 is provided for the retrieval of the sheath 86 from within the gastrointestinal tract. The release line 83 and the retrieval line 84 may be pulled to respectively allow the opening of the sheath 86 and the retrieval of the sheath 86.

Fig. 4 is an embodiment of the delivery device 10. An implant is attached thereto by an auxiliary medical device 140. The snare 90 of the delivery device can be seen to engage an auxiliary medical device of an implant 140. A flexible line 91 is shown which holds the connection element 110 with a looped attachment element 92 forming a snare 90. The connection element is formed as a dumbbell having two thicker portions 111' and 111‴ which are formed as silicone balls connected by a middle portion 112. The connection element 110 is formed integrally with a retrieval tail 170 of the auxiliary medical device 140. The snare 90 is indirectly connected to the elongate shaft 40 of the delivery device via the auxiliary medical device 140. The position and/or orientation of the implant can be adjusted by pulling on snare 90 which causes a movement (e.g. bending) of the auxiliary medical device 140 and of the shaft 40. Operation of the release line 183 releases the implant from the auxiliary medical device. The snare 90 remains attached to the connection element 110 which in turn remains connected to the auxiliary medical device 140 once the release line has been actuated and the implant is free from the auxiliary medical device 140. The snare 90 is then pulled to retrieve the auxiliary medical device and connection element from within the patient.

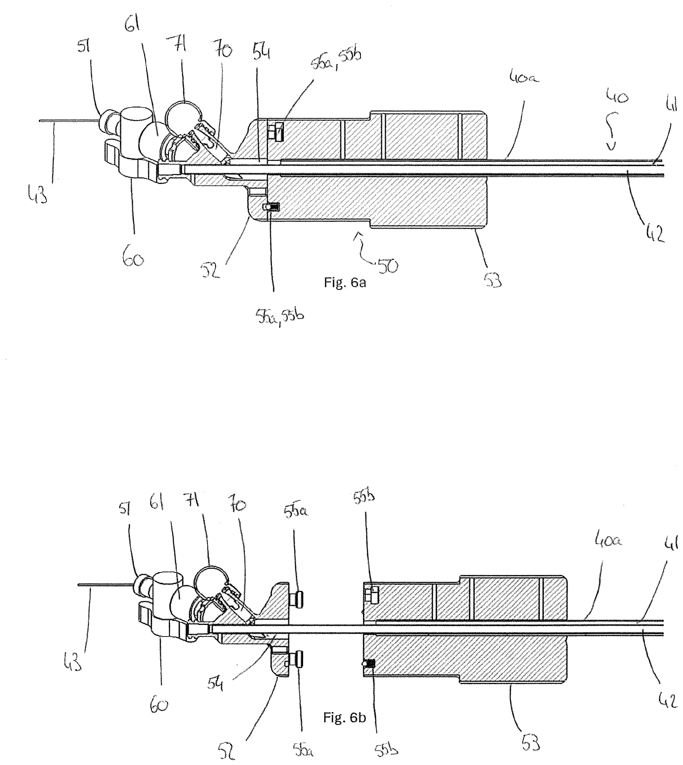

Figs. 5a and 5b illustrate an embodiment of handle 50 of the delivery system 10 having a detachable portion 52. Fig 5a depicts the handle 50 having the detachable portion 52 attached to the handle main body 53. The detachable extremity 52 is at the proximal end of the handle 50 and is connected to the handle body 53 by a locking system 55a, 55b (see fig. 5b and figs. 6a-b). The detachable extremity 52 comprises the port 70 for the insertion of a tool (not shown) within the working channel 41 (see fig. 6a-b) of the delivery system. At the other extremity, the handle body 53 is attached to the proximal end 40a of the elongated main shaft 40 of the delivery system. The handle comprises a first channel 50a extending from an opening 51 at the detachable extremity 52. A line 43 has been inserted through the opening 51 of the handle 50. The line 43 can be for example, an inflation line for inflation of an anchor of the implant or a release line for releasing an implant sheath. Here, the line 43 is introduced into the delivery device 10 through a second port 61. The second port 61 has an actuator configured to allow operation of the line 43.

Fig 5b illustrates the handle 50 of Fig. 5a wherein the detachable portion 52 has been detached from the handle body 53. In this exemplary case, the detachable extremity 52 has been detached for the removal of a deployer 42. The detachable portion comprises a lock component 55a of a locking system 55a, 55b configured to securely connect the detachable portion 52 of the handle 50 to the handle main body 53. The deployer 42 is attached to the detachable extremity 52 and extends into the handle main body 53 through an opening (not shown).

The line 43 can be seen to extend out of the detachable portion 52 and runs parallel to the deployer 42. The line 43 also extends into the handle main body 53 through the same opening as the deployer 42.

Fig. 6a corresponds to a longitudinal section view of the handle of fig. 5a. Corresponding reference numerals will not be redescribed for clarity. Here we can see that the handle 50 comprises a channel 54. The channel 54 of the handle extends within the detachable portion 52 and the handle main body 53 and is in fluid communication with a working channel 41 of elongate shaft 40. The elongate main shaft 40 can be seen to be partly housed in the channel 54 of the handle 50. The deployer 42 extends from the detachable portion 52 of the handle through the channel 54 of the handle and further extends within the working channel 41 of the shaft 40. The detachable portion 52 of the handle and the handle main body 53 are secured together by a locking system 55a,55b in a locked position.

Fig. 6b corresponds to a longitudinal section view of the handle of fig. 5b. Corresponding reference numerals will not be redescribed for clarity. The detachable portion 52 can be seen to have a first lock component 55a of the locking system and the handle main body 53 can be seen to have a second locking component 55b of the locking system. Here the first and second lock components 55a; 55b have been disengaged from one another to detach the detachable portion 52 of the handle from the main body 53 of the handle. The lock components 55a; 55b can be for example complementary elements of a rotary lock that are configured to engage one another by rotation of one or the other of the detachable portion 52 or the handle main body 53 with regard to the other. As a further example, the lock components 55a; 55b may be a twist pin lock having a pin which twistingly engages a complementary pinhole and groove. In this case, the pin of the lock is inserted into the lock groove via the pinhole and then twisting one or the other of the of the detachable portion 52 or the handle main body 53 with regard to the other moves the pin along the groove into a locked position which locks the two handle portions together. To unlock the twist pin lock, the two handle portions are again twisted, generally in the opposite direction compared to the twist direction for locking the portions of the handle together. This again causes the pin to slide along the groove until it reaches the pinhole and therefore the unlocked position where the two portions of the handle can be decoupled.

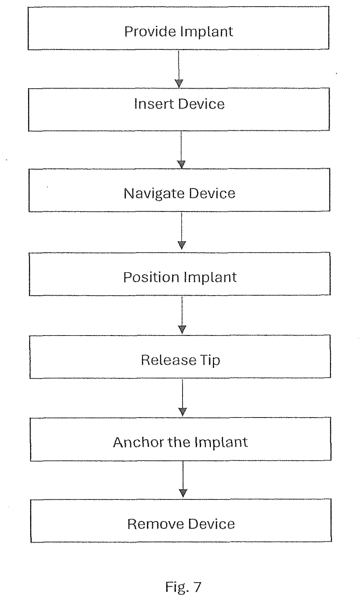

Fig. 7 illustrates the steps of a method of delivering an implant to the gastrointestinal tract of a patient using the delivery system 10. First an implant is coupled to the implant holder of the delivery system. The delivery system 10 is then inserted within the gastrointestinal tract of the patient. The delivery system is navigated such that the implant is positioned at, or near, to a target site, preferably at the pyloric sphincter of the patient. The position of the implant may, when required, be changed by actuation of a release line. To deliver the implant at the target site, the deployer 42 translates within the working channel 41 to advance the detachable tip along the gastrointestinal tract. This may additionally deploy the implant longitudinally within the intestine. The releasable connector of the deployer is then actuated such that the atraumatic tip is released from the deployer. This also releases the distal end of the implant such that it may extend from an insertion configuration'to a longitudinally fully extended state. An additional step of injecting a fluid through the sleeve may therefore be provided to achieve this. The method has a further step of anchoring the implant at the target site by deploying a duodenal and pyloric anchor. The anchors may be deployed by the removal of the implant sheath. This is achieved by actuation of the release line (i.e. by pulling) such that the sheath opens thus releasing the anchors. The duodenal anchor may self-deploy due to the elastic properties of the material used. The gastric anchor is a balloon anchor which is deployed by the injection of an inflation fluid via an inflation device thus inflating the anchor. The method comprises a final step of removing the delivery device. The removal is initiated with the retraction of the deployer and implant support along the elongate main shaft whilst the implant holder stays connected to the implant. This can be achieved by first disconnecting a detachable portion of the handle from the handle body followed by a pulling of the detached portion of handle which is attached to the deployer and support. The implant holder maintains the implant in the implanted position whilst the deployer and support are removed. Once the deployer and implant holder support have been removed, the implant holder is disengaged from the implant anchor and the elongate main shaft and implant holder attached thereto are pulled from the gastrointestinal tract. The retrieval of the delivery device in such a manner helps avoid the compromising of the deployed position of the implant during the removal.

Figs. 8-12 show similar components of the delivery device 10 and auxiliary medical device 140 as previously described in figures 1-6. The same reference numerals are not redescribed for clarity.

Fig. 8 depicts a delivery device comprising a similar handle 50 to the one shown in Figs. 5a-b and Figs. 6a-b. Here we can see a flexible line 91 of a snare which extends through the handle 50. The snare runs parallel to the elongated main shaft 40 of the delivery device from a proximal end 40a toward a distal end 40b. The implant holder 20 of the delivery device can be seen arranged at the distal end 40b of the main shaft 40. Adjacent to the implant holder 20, the flexible line 91 is attached via a looped attachment element 92 to the connection element 110 of an auxiliary medical device 140. A release line 183 of the auxiliary medical device 140 can be seen extending out from the implant holder 20 and threaded along the length of the auxiliary medical device 140. The auxiliary medical device 140 is shown in an exploded view without a crimping line, but the skilled person will understand that the auxiliary medical device 140 may be wrapped around an implant to crimp it (see Fig. 4) using a crimping line separate from the release line. The delivery device comprises an inflation line 22 for inflation of an implant anchor (not shown). The inflation line 22 extends from the end of the implant holder 20 and is configured to connect with an implant anchor when the implant is supplied to the delivery device 10. The implant holder 20 comprises a support 21 on which the implant (not shown) may be positioned. The support 21 extends within the elongate main 40 of the delivery device 10 and further extends distally from the implant holder 20. The support may be connected to the detachable portion of the main shaft. The support 21 has a lumen which can be seen in this embodiment to accommodate the deployer 42. Here, the deployer 42 extends out of the lumen of the support 21 and is attached at its distalmost end to the atraumatic tip 30 of the delivery device 10. The atraumatic tip 30 and the deployer 42 are connected to one another via the releasable connector 31. The atraumatic tip comprises an opening 32 through which a line or a guidewire may be passed (not shown).

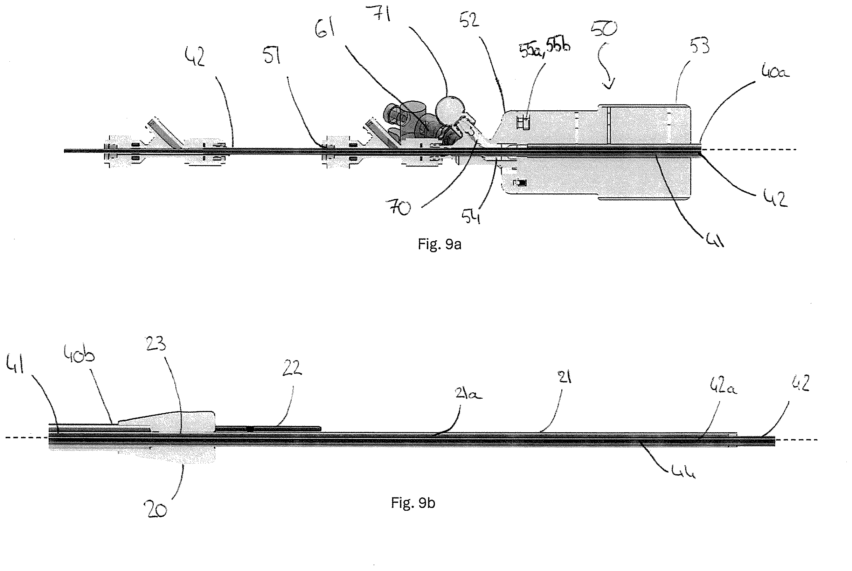

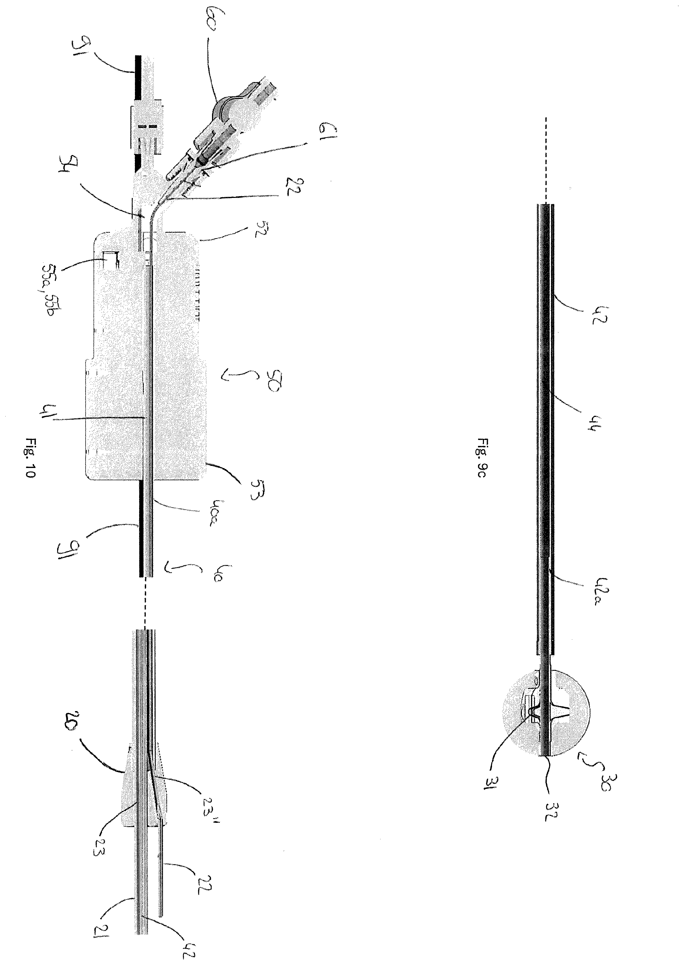

Figs. 9a-9c show a longitudinal section view of the handle 50 (Fig. 9a), the implant holder 20 (Fig. 9b) and the deployer 42 with the atraumatic tip 30 (Fig. 9c) of the delivery device 10. Here, we can see that the deployer 42 passes the opening 51 of the handle 50 into the channel 54 of the handle and enters the channel 41 of the elongated main shaft 40. Fig. 9b shows the support 21 extending from within the elongated main shaft 40 and passing within a channel 23 of the implant holder 20. The implant holder channel 23 is in communication with the channel 41 of the main shaft 40. The support 21 has a lumen 21a configured to house at least the deployer 42. Thus, the deployer 42 enters the device though opening 51, passes through channel 54, enters and extends along the channel 41 of the main shaft 40, then enters and runs along the channel 21a of the support 21 until it exits the support 21. Fig. 9c shows the deployer 42 having a lumen 42a which houses an inner shaft 44. The inner shaft 44 carries the releasable connection 31 and can in turn house a release mechanism (not shown) for operating the releasable connection 31. The inner shaft may further house a line or a guidewire (not shown). Thus, the delivery device 10 concentrically has from the outermost to innermost element: a handle 50, an elongated main shaft 40, support 21, deployer 42 and inner shaft 44, optionally a guidewire.

Fig. 10 also shows a sectional view of the handle 50 and implant holder 20 of the delivery device 10. Here, the inflation line 22 can be seen to extend from an inflation port 61 into the channel 54 of the handle 50 and passes into the channel 41 of the elongated main shaft 40. At the distal end 40b of the elongated main shaft 40 the inflation line 22 enters into the implant holder 20 and exits the implant holder 20 via a second channel 23'- of the implant holder 20 whilst the support 21 extends along the first channel 23 of the implant holder 20.

The inflation of the implant can be operated using the actuator 60 which is configured to open and close a valve (not shown) of the inflation port 61 to allow passage of a fluid for inflating the implant anchor when connected to the inflation line 21.

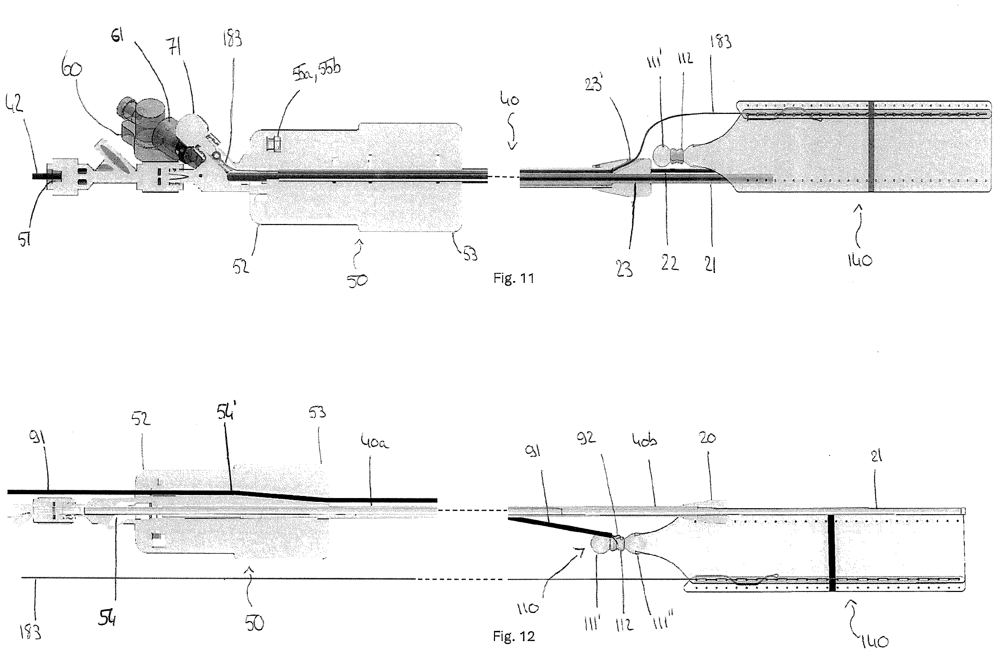

Fig. 11 shows a further sectional view of the handle 50 and implant holder 20 of the delivery device 10. The release line 183 of the auxiliary device 140 can be seen to extend from a port 70 of the delivery device 10 into the channel 54 of the handle 50 and extend along the channel 41 of the elongated main shaft 40. The port 70 comprises a knob 71 which closes and opens the port 70. The knob 71 is attached to the release line 183 of the auxiliary medical device 140. The release line 183 may for example be attached to the knob by wrapping around a protrusion of the knob which is housed within the port. As a further example, the release line 183 could be attached to the knob by an adhesive. The release line 183 can then be operated by the removal of the knob 71 from the port 70. The release line 183 exits the delivery device via a further channel 23" of the implant holder 20 and is threaded along the length of the auxiliary medical device 140. The auxiliary medical device 140 is arranged in a region adjacent to the inflation line 22 and the support 21 both of which extend from the implant holder 20.

Fig. 12 shows a sectional view of the handle 50 and implant holder 20 of the delivery device 10. Here, the flexible line 91 can be seen passing through a second channel 54' of the handle 50. The flexible line 183 exits the handle, runs substantially parallel to the main shaft 40 and attaches to the auxiliary medical device 140 via the attachment element 92. The attachment element 92 is looped around a connection element 110 of the auxiliary medical device 140. The release line 183 can be seen here to not pass though the delivery device 10 but rather extends parallel to the device 10. Thus, the release line 183 can be operated independent of the operating of the delivery device 10.

## Claims

1. A medical device, (10), preferably a delivery system for introducing and/or delivering an implant within the gastrointestinal tract and/or an endoscope, comprising an elongated main shaft (40), further comprising an attachment element, preferably a snare, for attaching to a line arranged on an outside of the elongated main shaft, the attachment element being positioned at or in vicinity of a distal portion of the elongated main shaft.

2. The medical device (10) according to claim 1, further comprising a line attached to the attachment element.

3. The medical device (19) according to any one of the preceding claims, comprising an implant holder (20) configured to hold an implant (80), wherein the implant holder (80) is coupled to, or is couplable to, the distal end (40b) of the elongated shaft (40), preferably wherein the implant holder is configured as an inner shaft arranged within and extending from the elongated main shaft.

4. The medical device (10) according to any one of the preceding claims, comprising a working channel (41) extending at least substantially the length of the elongated main shaft (40), preferably configured to house at least a deployer (42) .

5. The medical device (10) according to claim 4, wherein an implant is compressed by a jacket, wherein the attachment element is arranged on the jacket, and preferably wherein the implant holder is positioned at a distal end of the elongated main shaft.

6. The medical device (10) according to claim 5, wherein the jacket is releasable, and wherein the line is adapted to remove the jacket when released.

7. The medical device (10) according to any on of the preceding claims, further comprising an atraumatic tip (30), preferably a detachable tip, arranged at a distal end or distally (20b) of the implant holder (20).

8. The medical device wherein (10) according to any one of the preceding claims, wherein attachment element (90) is any one or more of: loop-like, lasso-like, or noose-like.

9. The medical device (10) according to any one of the preceding claims, further comprising at least one port (70), arranged at, or near, a handle (50), optionally in communication with the working channel (41).

10. A medical device (10), preferably a medical device according to any one of the preceding claims, preferably a delivery device and/or and endoscope, comprising an elongated main shaft (40) having a handle (50) arranged at, or near, a proximal end of the shaft (40a), wherein the handle (50) is at least partly detachable, preferably wherein the handle (50) comprises a detachable portion (52).

11. The medical device (10) according to claim 10, further comprising a deployer (42), said deployer (42) being configured to translate at least substantially the length of a working channel (41) for deploying an implant (80).

12. The medical device (10) according to claim 11, wherein at least one of the deployer and/or a support (21) arranged in the main shaft (40) is attached to the detachable portion of the handle, and wherein the deployer can be removed through the working channel by removal of the detachable handle.

13. The medical device (10) according to any claim 10 to 12, wherein the atraumatic tip (30) is coupled to a distal end of the deployer (42) by a releasable connection (45).

14. The medical device (10) according to any one of the preceding claims, wherein the attachment element (90) is configured to at least partly attach and/or at least partly encircle any one or more of: the elongated main shaft (40), an implant holder (20), an implant (80) or an implant sheath (86) for positioning of an implant (80) at a target site.

15. Method of delivering an implant (80), optionally using a medical device according to any one of the preceding claims, the method comprising one or more:
- providing an implant (80), optionally a gastro-intestinal sleeve, preferably a gastrointestinal sleeve comprising at least one anchor (81; 82), coupled to the implant holder (20),
- inserting a delivery system (10) within the gastrointestinal tract of a patient, preferably within the duodenum;
- steering a distal portion of the delivery device by pulling on a line which is attached to said distal portion via an attachment element, and/or releasing the implant from a jacket and removing the jacket by pulling on the line;
- optionally advancing the implant (80) distally via a deployer (42) until the implant (80), preferably the sleeve (85), is longitudinally deployed;
- optionally releasing the atraumatic tip (30) from the delivery device,
- optionally transitioning the implant (80), preferably a sleeve (85), from an circumferentially compressed configuration to a circumferentially deployed configuration,
- optionally actuating an anchor deployer for deploying at least one implant anchor (81; 82) of the implant.

16. The method according to claim 22 to 24, further comprising at least one of:
- actuating the anchor deployer for passively deploying a first implant anchor (81; 82),
- actuating the anchor deployer for actively deploying a second implant anchor (81; 82),
- operating a snare (90) for positioning an implant (80) relative to a target site,
- using an extraction mechanism, optionally by pulling motion, to retrieve an implant sheath (86).
